# EUROPÄISCHE PATENTSCHRIFT

(11) **EP 0 775 693 B1**
(45) Veröffentlichungstag und Bekanntmachung des Hinweises auf die Patenterteilung: **13.06.2001**
(21) Anmeldenummer: 96118042.9
(22) Anmeldetag: 11.11.1996
(51) Int. Cl.: C07C 319/20, C07C 323/36

(54) **Verfahren zur Herstellung von 2-Amino-6-chlorphenyl-isopropylsulfan**
Process for the preparation of 2-amino-6-chlorophenyl-isopropylsulfide
Procédé pour la préparation de 2-amino-6-chlorophényl-isopropylsulfure

(30) Priorität: 22.11.1995 DE 19543475
(43) Veröffentlichungstag der Anmeldung: 28.05.1997
(73) Patentinhaber: BAYER AG, 51368 Leverkusen (DE)
(72) Erfinder: Hagedorn, Ferdinand, Dr., 51381 Leverkusen (DE); Kiel, Wolfgang, Dr., 51519 Odenthal (DE); Fiege, Helmut, Dr., 51373 Leverkusen (DE)

(56) Entgegenhaltungen:
- WO-A-96/11906
- DE-A- 3 528 033
- FR-A- 1 489 916
- FR-A- 2 330 669
- J. HETEROCYCL. CHEM., Bd. 22, Nr. 5, 1985, Seiten 1345-1348, XP000616259 C. CORRAL ET AL:
- J. MED. CHEM., Bd. 34, Nr. 2, 1991, Seiten 675-687, XP000616258 H. INOUE ET AL:
- TETRAHEDRON, Bd. 20, Nr. 2, 1964, Seiten 177-187, XP000616256 K. PILGRAM ET AL:
- BULL. CHIM. SOC. DE FRANCE, 1951, Seiten 621-626, XP000616273 R. SPECKLIN ET AL:

## Beschreibung

Die vorliegende Erfindung betrifft ein Verfahren zur Herstellung von 2-Amino-6-chlorphenyl-isopropylsulfan in hohen Ausbeuten und Selektivitäten.

Substituierte Anilinosulfane sind Zwischenprodukte zur Herstellung von Pflanzenschutzmitteln (siehe z.B. EP-A2 - 0 306 222).

WO-96/11906 (veröffentlicht am 25.4.96) beschreibt die Herstellung von 2-Amino-6-chlorphenyl-isopropylsulfan durch Katalytische Hydrierung.

Es ist bekannt, dass man chlorfreies 2-Aminophenyl-n-propylsulfan durch katalytische Reduktion von 2-Nitrophenyl-n-propylsulfan herstellen kann (siehe Il. Farmaco, Ed. Sci. 12, 206 bis 217 (1957)). Die Reaktionsbedingungen sind jedoch ungünstig und die Ausbeuten mit rund 75 % recht niedrig.

Bei der katalytischen Reduktion von chlorsubstituierten Nitroaromaten erhält man chlorsubstituierte Aniline häufig nur in relativ geringen Selektivitäten, da dann auch eine hydrogenolytische Abspaltung von Chlor unter Bildung von entchloriertem Anilin erfolgt (siehe Chem. Ind. 1994, (53), S. 103 bis 109). Gemäß der EP-A1 - 0 409 709 wird bei dieser Reaktion die Tendenz zur hydrogenolytischen Abspaltung von Chlor durch Zusatz von Schwefelverbindungen, z.B. Thioharnstoff, erniedrigt. Der Zusatz von Schwefelverbindungen führt natürlich zu einer weiteren Komponente im Reaktionsgemisch, was dessen Aufarbeitung verkompliziert.

Beim Einsatzmaterial für das erfindungsgemäße Verfahren sind nicht nur Chlorsubstituenten vorhanden, sondern auch Sulfangruppen, die grundsätzlich ebenfalls hydrogenolytisch abspaltbar sind. Bei der Herstellung von 2-Amino-6-chlorphenylalkylsulfanen durch katalytische Hydrierung ist deshalb mit Selektivitäts- und Ausbeuteminderungen durch die Abspaltung von Chloratomen und/oder Sulfangruppen zu rechnen.

Es wurde nun ein Verfahren zur Herstellung von 2-Amino-6-chlorphenyl-isopropylsulfan gefunden, das dadurch gekennzeichnet ist, dass man 2-Chlor-6-nitrophenyl-isopropylsulfan ohne Zusatz einer weiteren Schwefelverbindung in Gegenwart von Methanol, bei Temperaturen im Bereich 50 bis 100°C und in Gegenwart von Raney-Nickel katalytisch hydriert.

Der Einsatzstoff 2-Chlor-6-nitrophenyl-isopropylsulfan ist literaturbekannt. In besonders vorteilhafter Weise kann man es aus dem entsprechenden 2,3-Dichlornitrobenzol durch Umsetzung mit einem Mercaptan der Formel (III) erhalten

H-S-R¹ (III),

in der
- R¹: Isopropyl bedeutet.

Man arbeitet dabei in Gegenwart einer Base, eines wässrigen Mediums und eines Phasentransferkatalysators bei 0 bis 100°C. Dieses Verfahren ist Gegenstand einer eigenen älteren Patentanmeldung.

Das Einsatzprodukt 2-Chlor-6-nitrophenyl-isopropylsulfan kann gelöst und/oder suspendiert in Methanol vorliegen.

Als Katalysator für das erfindungsgemäße Verfahren verwendet man Raney-Nickel. Besonders bevorzugt sind die Typen Raney-Nickel 55 und Raney-Nickel 37-1. Auch Legierungen des Raney-Nickels, z.B. Raney-Nickel-Eisen oder Raney-Nickel-Kobalt, können eingesetzt werden.

Die Katalysatormenge kann in einem weiten Bereich variiert werden. Beispielsweise kann man 0,5 bis 25 g Katalysator pro mol zu reduzierender Nitroverbindung einsetzen, wobei sich diese Gewichtsangabe auf feuchtes Katalysatormaterial bezieht.

Der anzuwendende Druck kann z.B. zwischen Normaldruck und 300 bar liegen. Bevorzugt sind 1 bis 150 bar, insbesondere 2 bis 100 bar.

Während der Durchführung des erfindungsgemäßen Verfahrens ist es zweckmäßig gut zu rühren.

Die Wasserstoffaufnahme ist im allgemeinen nach 30 Minuten bis 5 Stunden beendet. Gegebenenfalls kann man dann das Reaktionsgemisch noch einige Zeit, z.B. 10 Minuten bis 1 Stunde, bei Reaktionsbedingungen halten. Das Reaktionsgemisch kann man z.B. so aufarbeiten, dass man nach Abkühlung und Druckentspannung den Katalysator, z.B. durch Filtration, abtrennt, ihn mit Methanol nachwäscht, die Filtrate vereinigt und das Lösungsmittel daraus abtrennt, z.B. durch Abziehen im Vakuum oder durch Destillation. Man erhält dann ein Produkt, das in den meisten Fällen für die Weiterverwendung genügend rein ist. Gegebenenfalls kann man es, z.B. durch Destillation, leicht in Reinheiten von über 99 % erhalten.

Es ist ausgesprochen überraschend, dass man erfindungsgemäß 2-Amino-6-chlorphenyl-isopropylsulfan ohne den Zusatz von Schwefelverbindungen in hohen Ausbeuten und Selektivitäten erhalten kann. Enthalogenierungen und Entschwefelungen finden nur in ganz untergeordnetem Ausmaß (im allgemeinen jeweils unter 0,5 %) statt.

### Beispiele

Die Prozentangaben sind Gewichtsprozente, soweit nichts anderes angegeben ist.

### Beispiel 1

34,7 g 2-Chlor-6-nitrophenyl-isopropylsulfan, 140,0g Methanol und 1 g Raney-Nickel 55 als Katalysator (wasserfeucht) wurden in einem 0,3 Stahlautoklaven vorgelegt. Nach Verdrängen der Luft mit Stickstoff und Wasserstoff wurde bei Raumtemperatur unter Rühren des Gemisches Wasserstoff bis zu einem Druck von 80 bar aufgedrückt, wobei bereits ein leichter Temperaturanstieg beobachtet wurde. Man erhitzte das Gemisch auf 60°C unter weiterem Rühren und Konstanthalten des Wasserstoffdrucks. Nach etwa 2 Stunden war die Wasserstoff-Aufnahme beendet. Das Gemisch wurde dann auf Raumtemperatur abgekühlt, der Wasserstoff entspannt, der Katalysator abgesaugt, mit Methanol gewaschen und die vereinigten Filtrate eingeengt. Rohausbeute: 29,6 g 2-Amino-6-chlorphenyl-isopropylsulfan mit einer Reinheit von 99,5 % (GC), entsprechend 97,5 % der Theorie. Der Gehalt an 2-Isopropylmercaptoanilin, entstanden durch hydrierende Enthalogenierung, betrug nur 0,1 %. Ferner wurden nur 0,1 % Verunreinigungen durch 3-Chloranilin, entstanden durch hydrierende Entschwefelung, gefunden.

### Beispiel 2

46,6 g 2-Chlor-6-nitrophenyl-isopropylsulfan, 160 ml Methanol und 5 g Raney-Nickel 55 (wasserfeucht, mit Methanol gewaschen) wurden in einen Stahlautoklaven gefüllt. Nach Spülen mit Stickstoff und Wasserstoff wurde das Gemisch unter Rühren auf 50°C erhitzt und unter Aufpressen von Wasserstoff bis maximal 2 bar hydriert. Nach 2,5 Stunden war die Wasserstoffaufnahme beendet. Das Gemisch wurde dann auf Raumtemperatur abgekühlt, der Wasserstoff entspannt, der Nickelkatalysator durch Absaugen abgetrennt und mit Methanol gewaschen. Nach dem Entfernen des Lösungsmittels erhielt man 39,9 g 2-Amino-6-chlorphenyl-isopropylsulfan in einer Reinheit von 97,8 % (GC), was 97 % der Theorie entspricht. An Verunreinigungen wurden nur 0,2 % 2-Aminophenylisopropylsulfan (entstanden durch Enthalogenierung) gefunden. Durch Destillation erhielt man bei einem Siedepunkt von 144°C bei 11 mbar reines, farbloses 2-Amino-6-chlorphenyl-isopropylsulfan mit einem Gehalt von über 99 %.

### Beispiel 3

In einem 0,7 1 Stahlautoklaven wurden 40 g 2-Chlor-6-nitrophenyl-isopropylsulfan, 160 g Methanol und 2,4 g Raney-Nickel 37-1 (methanolfeucht) vorgelegt. Nach Spülen mit Stickstoff wurden 70 bar Wasserstoff aufgedrückt und der Autoklaveninhalt unter Rühren auf 100°C erhitzt. Der Wasserstoffdruck wurde auf 80 bar erhöht. Nach 50 Minuten war die Wasserstoffaufnahme beendet. Das Gemisch wurde noch 30 Minuten bei Reaktionsbedingungen gehalten. Nach dem Abkühlen, dem Entspannen des Wasserstoffs und dem Abtrennen des Katalysators wurde das Lösungsmittel abdestilliert. Man erhielt das rohe 2-Amino-6-chlorphenyl-isopropylsulfan bereits als 99 %iges Produkt (GC), das nur 0,2 % 2-Aminophenyl-isopropylsulfan (entstanden durch Entchlorierung) und nur 0,3 % m-Chloranilin (entstanden durch Entschwefelung) enthielt. Die Ausbeute betrug 34,5 g.

## Patentansprüche

1. Verfahren zur Herstellung von 2-Amino-6-chlorphenyl-isopropylsulfan, dadurch gekennzeichnet, dass man 2-Chlor-6-nitrophenyl-isopropylsulfan ohne Zusatz einer weiteren Schwefelverbindung in Gegenwart von Methanol, bei Temperaturen im Bereich 50 bis 100°C und in Gegenwart von Raney-Nickel katalytisch hydriert.

## Claims

1. Process for the production of 2-amino-6-chlorphenylisopropyl sulfane, characterised in that 2-chloro-6-nitrophenylisopropyl sulfane is catalytically hydrogenated without the addition of a further sulfur compound in the presence of methanol at temperatures in the range from 50 to 100°C and in the presence of Raney nickel.

## Revendications

1. Procédé pour la préparation du 2-amino-6-chlorophényl-isopropylsulfane, caractérisé en ce que l'on soumet le 2-chloro-6-nitrophényl-isopropylsulfane à hydrogénation catalytique en présence de nickel de Raney, à des températures dans l'intervalle de 50 à 100°C et en présence de méthanol, sans adjonction d'un autre dérivé sulfuré.
